# EUROPEAN PATENT SPECIFICATION

(11) **EP 2 233 476 B1**
(45) Date of publication and mention of the grant of the patent: **28.08.2013**
(21) Application number: 10005763.7
(22) Date of filing: 09.12.2005
(51) Int. Cl.: C07D 307/46, C07D 307/42, C08L 9/08

(54) **Preparation of an ether of 2,5-(hydroxymethyl)furfural (HMF) from a fructose source, an alcohol solvent and an acid catalyst**
Herstellung eines Ethers des 2,5-(Hydroxymethyl)furfurals (HMF) ausgehend von einer Fructose-Quelle, eines Alkohol-Lösungsmittels und eines sauren Katalysators
Préparation d'un éther de 2,5-(hydroxymethyl)furfural (HMF) à partir d'une source de fructose, un solvant d'alcool et un catalysateur acidique

(30) Priority: 10.12.2004 US 635406 P; 02.03.2005 US 70063
(43) Date of publication of application: 29.09.2010
(62) Divisional of application: 05853500.6
(73) Proprietor: ARCHER-DANIELS-MIDLAND COMPANY, Decatur, IL 62525 (US)
(72) Inventor: Sanborn, Alexandra J., Lincoln IL 62656 (US)
(74) Representative: von Kreisler Selting Werner

(56) References cited:
- BROWN DC W ET AL: "Dehydration reactions of fructose in nonaqueous media" JOURNAL OF CHEMICAL TECHNOLOGY AND BIOTECHNOLOGY, BLACKWELL SCIENTIFIC PUBLICATIONS. OXFORD, GB, vol. 32, no. 10, 1 January 1982 (1982-01-01), pages 920-924, XP009095767 ISSN: 0268-2575
- MOYE C J ET AL: "Reaction of ketohexoses with acid in certain non-aqueous sugar solvents" JOURNAL OF APPLIED CHEMISTRY, SOCIETY OF CHEMICAL INDUSTRY. LONDON, GB, vol. 16, no. 7, 1 January 1966 (1966-01-01), pages 206-208, XP009067960
- WEIDENHAGEN R ET AL: "Ueber die Darstellung von Alkoxymethylfurfurolen und Laevulinsaeurealkylestern aus Kohlenhadraten. 2. Mitteilung//Preparation of alkoxymethylfurfuraldehedes and alkyl levulates from carbohydrates. II" ZEITSCHRIFT DER WIRTSCHAFTSGRUPPE ZUCKERINDUSTRIE, REICHSWIRTSCHAFTSKAMMER / WIRTSCHAFTSGRUPPE ZUCKERINDUSTRIE, DE, vol. 85, 1 January 1935 (1935-01-01), pages 131-136, XP009095653 ISSN: 0373-0336

## Description

### Field Of The Invention

Improved methods of producing chemical compounds are included herein. The dehydration reaction of common carbohydrates to form commercially important compounds, furan derivatives, and methods of optimizing the reactions to efficiently synthesize the products, as well as improved methods of purification are included herein.

### Background Of The Intention

2,5-(Hydroxymethyl)furaldehyde, also known as 2,5-(hydroxymethyl)funfural (HMF), has many important industrial and commercial applications, largely due to its many functional groups and ability to serve as a precursor in many polymerization reactions. HMF, for example, is a suitable starting source for the formation of various furan monomers required for the preparation of non-petroleum-derived polymeric materials. HMF, as well as other 2,5-disubstituted furanic derivatives, also has great potential for use in the field of intermediate chemicals from regrowing resources. Also due to its various functionalities, HMF may be used to produce a wide range of products, including, but not limited to, polymers, solvents, surfactants, pharmaceuticals, and plant protecting agents. HMF is shown in the structure below:

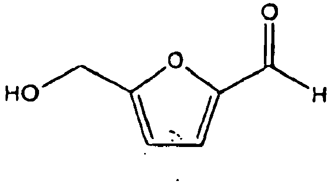

The use of HMF and other furfural derivatives may be compared with the use of corresponding benzene-based macromolecular compounds. In order to be cost-effective and compete in this market, HMF must be able to be produced at competitive prices. The production of HMF has been studied for years, but an efficient and cost-effective method of producing HMF in high yields has yet to be found. HMF is primarily produced from the dehydration reaction of a carbohydrate compound, particularly monosaccharides, including glucose and fructose. Complications arise from the rehydration of HMF after the dehydration occurs, which often yields the by-products of levulinic acid, and formic acid. Another competing side reaction is the polymerization of HMF and/or fructose to form humin polymers.

Hexoses are the preferred carbohydrate source from which HMF is formed. Fructose is the preferred hexose used for the dehydration reaction to form HMF. This is in part because fructose has been shown to be more amendable to the dehydration reaction to a form HMF. Fructose is shown by the structures below:

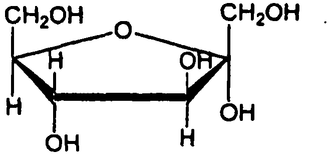

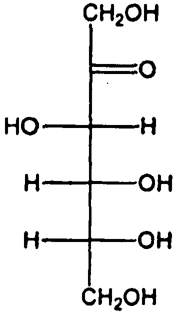

Fructose however, is more expensive than other hexoses, such as glucose (dextrose), and maltose, for example. Early processes and procedures for the production of HMF concentrated on the use of crystalline fructose, but its widespread use is prevented by its high cost. Other sources of fructose, including high-fructose corn syrup (HFCS), have been used to produce HMF and other furan derivatives. Szmant and Chundury used high fructose corn syrup as a starting material in forming HMF, as disclosed in a 1981 article in J. Chem. Tech. Biotechnol., 31, (pgs. 135-145). Szmant uses a variety of carbohydrates as starting material, but designs reaction conditions specific to each fructose source. Szmant, for example, uses a boron trifluoride catalyst (BF₃ Et₂O) with DMSO as a solvent in the conversion of HFCS to HMF, but utilizes different catalyst/solvent combinations with different starting materials. Use of BF₃ Et₂O as a catalyst is not economically practical since it cannot be recovered and re-used. Furthermore, Szmant requires the use of a Pluronic emulsifier to suppress foaming. Szmant also requires bubbling of nitrogen to suppress oxidation. Still further, Szmant requires the use of DMSO as a solvent, which is not easily separable from the HMF product, and therefore creates difficulties with product recovery. It is very desirable, therefore, to develop an industrially practicable process for producing HMF in high purity.

U.S. Patent No. 6,706,900 to Grushin et al. (Grushin '900) also discloses the dehydration of fructose in the form of high-fructose corn syrup, to form HMF as an intermediate; but this process is performed in the context of forming diformylfuran, also known as 2,5-dicarboxaldehyde (DFF). The reaction proceeds in an aqueous environment, and the HMF that is formed is not isolated from the reaction mixture, but rather is directly converted to DFF without an isolation step. The reaction conditions of Grushin '900 are therefore not constrained by considerations of product yields of HMF, as it is formed as an intermediate that is not isolated as a product. More importantly from a practical commercial standpoint, Grushin '900 is not constrained by considerations of isolating HMF from the product mixture. An efficient method for producing HMF in desirable yields and sufficiently high purity from a natural and industrially convenient fructose source that may include other mixed carbohydrates has yet to be found.

Water has in the past been used as a solvent of choice in dehydration reactions forming HMF because of the solubility of fructose in water. Aqueous conditions, however, have proven to deleteriously affect the dehydration reaction of fructose to HMF in a variety of ways. Aqueous conditions have led to decreased yield of HMF as low selectivity for the dehydration reaction has been demonstrated. Furthermore, solvation of protons in water highly reduces the catalytic activity for the dehydration reaction. Low selectivity of the dehydration reaction simultaneously leads to increased polymerization reactions and humin formation, which also interfere with the synthesis of HMF.

In an attempt to solve such problems associated with aqueous systems, one proposed solution involves an improvement by simultaneously extracting HMF after the dehydration reaction. A similar attempt to improve yields involves the adsorption of HMF on activated carbon. The key factor in these processes is a rapid removal of HMF from the acidic medium in which it is formed. However, these systems generally suffer from high dilution or partially irreversible adsorption of HMF.

In another attempt to solve the problems of aqueous systems, an organic solvent may be added to the aqueous solution, such as, for example, butanol or dioxane. Such systems, however, present a difficulty in that rehydration of HMF is common and ether formation of HMF occurs with the solvent if alcohols are employed. High yields of HMF, therefore, were not found with the addition of these organic solvents. In a further attempt to provide an adequate solvent system, aqueous solvent mixtures and anhydrous organic solvents have also been employed to ensure favorable reaction conditions. Examples of anhydrous organic solvents used include dimethylformamide, acetonitrile, dimethylsulfoxide, and polyethylene glycol.

Dimethylsulfoxide (DMSO), for example, has been extensively studied and employed as a solvent in the dehydration reaction to form HMF. Improved yields of HMF have been reached with ion exchangers or boron trifluoride etherate as a catalyst, and even without any catalyst. DMSO presents a problem, however, in that recovery of HMF from the solvent is difflcult.

Furthermore, although dehydration reactions performed in solvents with high boiling points, such as dimethylsulfoxide and dimethylformamide, have produced improved yields, the use of such solvents is cost-prohibitive, and additionally poses significant health and environmental risks in their use. Still further, purification of the product via distillation has not proven effective for a variety of reasons. First of all, on long exposure to temperatures at which the desired product can be distilled, HMF and impurities associated with the synthetic mixture tend to be unstable and form tarry degradation products. Because of this heat instability, a falling film vacuum still must be used. Even in use with such an apparatus however, resinous solids form on the heating surface causing a stalling in the rotor, and the frequent shutdown resulting therefrom makes the operation ineffcient.

Catalysts may also be used to promote the dehydration reaction. Some commonly used catalysts include cheap inorganic acids, such as H₂SO₄, H₃PO₄. HCl, and organic acids such as oxalic acid, levulinic acid, and p-toluene sulfonic acid. These acid catalysts are utilized in dissolved form, and as a result pose significant difficulties in their regeneration and reuse, and in their disposal. In order to avoid these problems, solid sulfonic acid catalysts have also been used. Solid acid resins, however, are limited in use by the formation of deactivating humin polymers on their surfaces under conditions taught by others. Other catalysts, such as boron trifluoride etherate, can also be used. Metals, such as Zn, Al. Cr, Ti, Th, Zr, and V can be used as ions, salts, or complexes as catalysts. Such use has not brought improved results. however, as yields of HMF have continued to be low. Ion exchange catalysts have also been used, but have also delivered low HMF yields under conditions taught by others, and further limit the reaction temperature to under 130°C.

Brown et al., Journal of Chemical Technology and Biotechnology, Vol. 32, No. 1, 1982, pages 920 to 924, describe dehydration reactions of fructose in non-aqueous media.

Moye et al., Journal of Applied Chemistry, Vol. 16, No. 7, 1966, pages 206 to 208, disclose the reaction of sucrose and fructose with acids at high temperatures in non-aqueous solvents.

Weidenhagen et al., Zeitschrift der Wirtschaftsgruppe Zuckerindustrie, Vol. 85, 1935, pages 131 to 136, describe the preparation of alkoxymethylfurfurols and levulinic acid alkyl esters from carbohydrates.

### Summary of the Invention

The object of the present invention is solved on the basis of claims 1 to 9.

Provided herein is a method of preparing an R-oxymethylfurfural ether of hydroxytmethylfurfural of the formula: 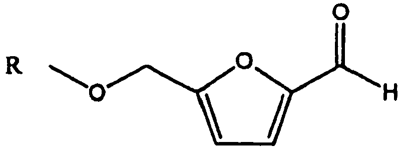 where R is selected from the group consisting of alkyl, cycloalkyl, allyl and aryl, comprising: (i) combining a fructose source, an R-OH solvent, and an acid catalyst to form a reaction mixture; (ii) heating said reaction mixture to a temperature and for a time sufficient to promote an acid-catalyzed dehydration reaction fructose in the fructose source and to form R-oxymethylfurfural in a product mixture; and (iii) Isolating the R-oxymethylfurfural from said product mixture.

In one embodiment the fructose source is high fructose corn syrup, and the method is performed under vacuum conditions. In a further embodiment the carbohydrate source is added gradually in a stepwise fashion once the reaction has been initiated, this entails the addition of two or more discrete aliquots over a specified period of time. In an additional embodiment, the mixed carbohydrate source comprises a first carbohydrate source in a first physical state, and a second carbohydrate source in a second physical state, wherein the first and second physical states are not the same, that is to say they are in different physical states. Suitable carbohydrate sources include, but are not limited to, a hexose, a pentose, fructose syrup, crystalline fructose, and, process streams from the crystallization of fructose.

Suitable mixed carbohydrate source may comprise any industrially convenient carbohydrate sources, such as corn syrup. The mixed carbohydrate sources include hexoses, fructose syrup, crystalline fructose, high fructose corn syrup, crude fructose, purified fructose, high fructose corn syrup refinery intermediates and by-products, process streams from crystallizing fructose or glucose, and molasses, such as soy molasses resulting from production of soy protein concentrate.

In one embodiment, after product isolation the ion-exchange resin catalyst may be rinsed with the organic solvent used to carry out the reaction to recover product contained within the resin. After the rinse, the ion-exchange resin catalyst may be reused in a subsequent reaction. In a further embodiment, after product isolation the ion-exchange resin may be rinsed with a second organic solvent to recover product contained with in the resin. After the rinse, the ion-exchange resin catalyst may be reused in a subsequent reaction.

In an embodiment, the purification of the product isolate may be performed by a process selected from the group consisting of short path distillation, thin film evaporation, wiped film evaporation, crystallization, and adsorption to an inert adsorbent. Adsorbents include silica, carbon, alumina, and other resins. A non-volatile flowing agent may be added to the product isolate to enhance separation. The non-volatile flowing agent may be chosen from the group consisting of polyethylene glycol, polyethylene glycol monoether, polyethylene glycol diether, and combinations thereof. In a further embodiment, the non-volatile flowing agent may be purified to a re-usable form after it has performed its role in the purification process. Such purification process may take place with the use of carbon as disclosed herein.

The present invention provides in embodiments:
(1) A method of preparing an R-oxymethylfurfural ether of hydroxylmethylfurfural of the formula: 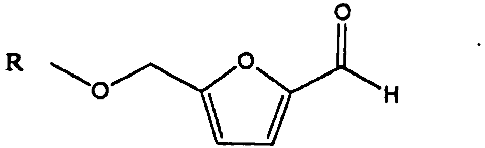 where R is selected from the group consisting of alkyl, cycloalkyl, allyl and aryl, comprising:
   (i) combining a fructose source, an R-OH solvent, and an acid catalyst to form a reaction mixture;
   (ii) heating said reaction mixture to a temperature and for a time sufficient to promote an acid-catalyzed dehydration reaction fructose in the fructose source and to form R-oxymethylfurfural in a product mixture; and
   (iii) Isolating the R-oxymethylfurfural from said product mixture.
(2) The method of embodiment (1) wherein the R-OH solvent is ethanol and the R-oxymethylfurfural is ethoxymethylfurfural.
(3) The method of embodiment (1) wherein the acid catalyst is an acidic ion exchange resin.

### Detailed Description of the Invention

Reusable or recyclable catalysts are preferred for use in the reaction, as they provide for increased efficiency, and economic and industrial feasibility. As used herein, the term "recyclable catalyst" refers to a catalyst which is not irreversibly expended as a result of the reaction. In other words, the catalyst may be used again. Examples of recyclable or reusable catalysts include solid acid catalysts, ion-exchange resins, zeolites, Lewis acids, clays, and molecular sieves. Stolid acid catalysts often comprise a solid material which has been functionalize to impart acid groups that are catalytically active. Solid acid catalysts may have a broad range of composition, porosity, density, type of acid groups and distribution of acid groups. Solid acid catalysts may be recovered and reused, optionally with a treatment to regenerate any activity that may have been lost in use. Some solid acid catalysts that may be used in the disclosed process include, but are not limited to Amberlyst 35, Amberlyst 36, Amberlyst 15, Amberlyst 131 (Rohm and Haas, Woodridge, IL), Lewatit S2328, Lewatit K2431, Lewatit S2568, Lewatit K2629 (Sybron Corp, Birmingham, NJ), Dianion SK104, Dianion PK228, Dianion RCP160, RCP21H, Relite RAD/F (Mitsubishl Chemical, White Plains, NY), and Dowex 50WX4 (Dow Chemical).

One example of a solvent that may be used is a polar solvent. The polar solvent maybe a polar aprotic solvent. Examples of possible solvents include. but are not limited to, 1-methyl-2-pyrrolidinone, dimethylacetamide, dimethylformamide, dimethyl sulfoxide, methyl ethyl ketone, methyl isobutylketone, acetonitrile, propionitrile, and combinations thereof.

By removing water as it is formed, side-reactions are thereby minimized, and an increased yield has been observed. Water removal may take place via evaporation. A rotary evaporation machine may be employed to promote water removal. The use of a rotary evaporator, or "rotovap," is well-known in the art. Water removal may also be carried out by evaporation from the reaction mixture and condensation as Ice or water on a cold finger or reflux condenser. Water may also be removed by distillation, including azeotropic distillation with a water-entraining solvent which may optionally be stripped of water and the water depleted solvent returned to the reaction vessel. A suitable distillation apparatus, such as a Barrett type receiver may also be employed. A water-absorbing material may also be used to remove water. Such materials are well-known in the art, and include molecular sieves.

In one embodiment, the reactions disclosed herein are performed at moderately high temperatures, typically in a range of from about 95° to about 125°C. In a further embodiment, the temperature range is from about 105° C to about 115° C. It is preferable to use temperatures below 200 degrees Celsius. The reactions disclosed herein typically occur in a time frame of from about one to about six hours. More typically, the reactions take from about two hours to about five and a half hours.

As used herein; the term "zeolite" refers to a hydrated silicate of aluminum and one or both of sodium and calcium. Examples include analcite, chabazite, heulandite, natrolite, stilbite, thomsonite, in either powder or pellet form. Commercial zeolites products inctude CBV 3024 and CBV 5534G (Zeolyst International), T-2665, T-4480 (United Catalysis, Inc), LZY 64 (Union Carbide), and H-ZSM-5 (PQ Corporation).

As used herein, Cornsweet 90 refers to a high fructose corn syrup product of commerce nominally containing 60% to 70% fructose. High fructose corn syrup refinery intermediate and by-product is a fructose-rich stream generated in a fractionation system positioned after an isomerization column in the production of high fructose corn syrup. A suitable process stream from crystallizing fructose is called "mother liquor" and comprises a solution of fructose in ethanol. Typically this process stream is about 24% solids, almost all of the solids being fructose, and contains about 60% ethanol. A similar mother liquor from glucose crystallization contains about 50% solids. Mixed carbohydrate sources can be obtained by blending carbohydrates, such as by adding crystalline fructose to high fructose corn syrup.

As used herein, "reaction yield* is calculated using the equation (moles of product/moles of starting material)*100. Product purity is reported on a weight percent basis.

As used in this equation, "starting material" refers to the fructose present in the carbohydrate source, mixed carbohydrate source, or other reactant for the particular dehydration reaction.

As used herein, the term "fructose source" refers to a material that comprises sucrose. Typical embodiments are solutions having at least 25% sucrose by solute weight, and which may include other materials such as other carbohydrate compounds. Preferably, the carbohydrate compounds are hexoses. The versatility of the reaction conditions provided herein allow an industrially convenient source to be used as the starting material, that is to say, the reaction is not limited to a particular carbohydrate source or to fructose of high purity.

Suitable fructose sources typically include high fructose corn syrup (HFCS) or any HFCS refining process stream that includes at least 25% sucrose. HFCS is typically commercially available in products comprising solutions having 42% to 95% fructose by solute weight which are typically sold for use as industrial scale sweeteners. The most economical embodiments of the invention use HFCS having about 90% sucrose by solute weight. However, less economical embodiment's invention can be practiced with sources having less sucrose by weight. To improve economic efficiencies, less pure sucrose sources can be conveniently blended with higher purity sucrose sources or even crystalline sucrose to achieve a solution having at least 25% sucrose by solute weight.

Optional neutralization of the product isolate is carried out by addition of a suitable alkali substance, such as a basic ion exchange resin, potassium hydroxide, or sodium hydroxide. This neutralization step allows for subsequent product recovery by distillation without heat-catalyzed degradation or polymerization, resulting in the elimination of tarry degradation products and resinous solids being formed in distillation. This neutralization step also allows for subsequent product recovery with a flowing agent without heat-catalyzed degradation or polymerization; resulting in the elimination of tarry degradation products and resinous solids being formed in distillation.

Purity was determined by ¹³C NMR and Proton NMR, in some cases by capillary GC, and in some cases by UV adsorption.

As used herein, the term "non-aqueous mixture" refers to a mixture comprising a non-aqueous solvent and at least one other component, wherein the content of the solvent is greater than the content of the at least one other component, as measured by volume. The at least one other component may comprise, without limitation, a water-containing substrate, such as HFCS, or an organic solvent. Non-aqueous solvents are usually measured by volume, and other components are usually measured by weight.

As used herein, the term "isolate" refers to the process of preservation of a material originally present in a product mixture after the product mixture has been subjected to a step to remove other material from the product mixture, as well as the isolated material resulting from the process. Examples of "other material* that is removed includes solid material, such as catalyst by methods including the processes of filtration, decantation, centrifugation, and washing. Filtration may be performed by one of the processes selected from the group comprising gravity filtration, vacuum filtration, and suction filtration.

The term "non-volatile flowing agent" as used herein refers to an inert material which, when added to a product mixture, aids in the recovery of the desired compound by distillation. In certain embodiments, the fugacity of the flowing agent is sufficiently low so that it will not volatilize as the target product is removed by evaporation.

It has been surprisingly found that HMF ethers may be synthesized using the methods of the present invention with slight variations. Generally, ethers may be formed from any R group, such as alkyl, cyctoalkyl, allyl, aryl. Such variations Include the introduction of alcohol having the appropriate constituent R group, such as, for example, ethanol (EtOH) where R is C₂H₅, as a polar solvent in either batch reactions or via column elution. This method would therefore comprise: i) combining materials comprising a fructose source, an alcohol solvent, and a catalyst to form a reaction mixture; ii) heating said reaction mixture to a temperature and for a time sufficient to promote an acid-catalyzed dehydration reaction of the fructose in the fructose source to form a product mixture; and iii) isolating an ether derivative from said product mixture. HMF ethers, such as ethoxymethylfurfural (EMF), are more stable than HMF because they lack the exposed hydroxyl group of HMF. EMF is shown in the structure below:

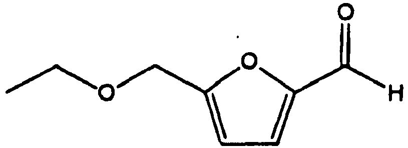

In an embodiment the fructose source is a HFCS. The use of a column in the synthetic process enables a continuous flow of heated fructose solution, thereby decreasing the amount of polymerization and by-product formation. Further distillation may also be performed to purify EMF from the product mixture. The use of column elution creates a continuous flow and is a fairly simple process that efficiently leads to a more stable product. The subsequent purification via distillation is also a simple process that is economically feasible. Furthermore, yields have been surprisingly high, in the range of 85-100%. Purification may also be used in the form of liquid or gas chromatography.

### Examples

The following are examples of the dehydration of a fructose source to a furan derivative or organic acid, as well as isolation and/or purification techniques to optimize product recovery of increased product yield.

### EXAMPLE 1

### PREPARATION OF EMF FROM FRUCTOSE IN BATCH MODE

A 500 mL round bottom flask equipped with a reflux condenser, temperature probe, and magnetic stir bar was charged with a solution of 30 g fructose (Aldrich), 225 mL HPLC grade ethanol (Aldrich), and 30 g of Amberlyst 131 resin (Rohm and Haas). Amberlyst 131 is a strongly acidic polymeric catalyst with a particle size of 0.7-0.8 mm and water content of 65%. The stirred mixture was heated to reflux for 24 hours. At this time, the slurry was filtered and the resin washed with ethanol to provide 174 mL of product isolate containing 5.4 g/L HMF and 61.6 g/L EMF.

### EXAMPLE 2

### PREPARATION OF EMF FROM FRUCTOSE VIA COLUMN ELUTION

A 100 mL glass liquid-chromatography column (2.54 cm I.D) was slurry packed in HPLC grade ethanol (EtOH) with Amberlyst 131 resin obtained from Rohm and Haas Company (Woodridge, IL). The resin was washed with 500 mL of EtOH. The final packed volume was 100 ml. The feed material consisted of 5 mL of a 20% solution of fructose in EtOH. The feed was then loaded on the resin column by gravity flow and fractions were eluted. The column was maintained at 60°C and elution at 0.6 mL/min. Table 2 summarizes the results of this study. A complete conversion of fructose to a mixture of HMF/EMF was achieved, with the major product being EMF.

**Table 2. Column Synthesis of EMF from Fructose using Amberlyst 131 Resin.¹**

| **Fraction #** | **Volume (mL)** | **Fructose (ppm)** | **HMF (ppm)** | **EMF (ppm)** |
|---|---|---|---|---|
| 2 | 8 | 0 | 0 | 0 |
| 5 | 13.6 | 0 | 0 | 0 |
| 7 | 21.6 | 0 | 0 | 294 |
| 9 | 32.1 | 262 | 370 | 1,862 |
| 11 | 40.1 | 79 | 420 | 2,613 |
| 13 | 48.1 | 134 | 364 | 4,451 |
| 15 | 57.1 | 119 | 794 | 6,008 |
| 17 | 65.6 | 120 | 615 | 6,385 |
| 19 | 73.6 | 0 | 308 | 4,293 |
| 21 | 82.1 | 0 | 0 | 1,488 |
| 24 | 94.1 | 0 | 0 | 276 |
| 26 | 102.1 | 0 | 0 | 60 |

| | | | | |
|---|---|---|---|---|
| 'Column was maintained at 60° C with a steady flow rate of 0.6 mL/mln. | | | | |

### EXAMPLE 3

### PREPARATION OF EMF FROM FRUCTOSE VIA COLUMN ELUTION

This example illustrates the effect of change in resin to Amberlyst 35 obtained from Rohm and Haas Company (Woodridge, IL). Amberlyst 35 is a macroreticular, strongly acidic, polymeric catalyst. The feed material was prepared and loaded on to the column by gravity flow as described in Example 2. The column was maintained at 60°C and the elution was carried out at 0.6 mL/min. A summary of this is provided in table 3. Nearly 85% of the starting fructose was converted into a mixture of HMF/EMF with the major product being EMF.

**Table 3. Column Synthesis of EMF from Fructose using Amberlyst 35 Resin.¹**

| **Fraction #** | **Volume (mL)** | **Fructose (ppm)** | **Ethyl Levulinate (ppm)** | **HMF (ppm)** | **EMF (ppm)** |
|---|---|---|---|---|---|
| 1 | 2.0 | 263 | 242 | 263 | 263 |
| 3 | 13.0 | 271 | 249 | 271 | 271 |
| 5 | 25.0 | 230 | 212 | 230 | 230 |
| 7 | 34.5 | 253 | 233 | 253 | 253 |
| 8 | 37.5 | 227 | 209 | 227 | 579 |
| 10 | 46.5 | 2,737 | 948 | 1,180 | 5,687 |
| 12 | 58.0 | 2,507 | 203 | 1,157 | 7,844 |
| 14 | 67.0 | 1,970 | 1,528 | 1,188 | 9,526 |
| 16 | 76.0 | 520 | 246 | 325 | 2,023 |
| 18 | 85.0 | 282 | 260 | 282 | 282 |
| 19 | 89.5 | 256 | 236 | 256 | 256 |
| 20 | 96.5 | 269 | 248 | 269 | 269 |

| | | | | | |
|---|---|---|---|---|---|
| Column was maintained at 60° C with a steady flow rate of 0.6 mL/min. | | | | | |

### EXAMPLE 4

### PROCESS FOR THE SYNTHESIS AND PURIFICATION OF EMF

Dehydration: Amberlyst 131 Wet (145 g) was dried in vacuum at 85°C for three days. This catalyst was combined with 117 g crystalline fructose and 468 g of 100% ethanol in a steel reactor. With stirring at 600 rpm, the reaction mixture was gradually heated to 110° C over 30 minutes. The temperature was maintained for 45 minutes, and then the reaction mixture was cooled to ambient temperature over 7 minutes. The catalyst was filtered from the red-black reaction mixture, and the reaction mixture was treated with a rotary evaporator under house vacuum to remove ethanol.

Distillation of EMF on Wiped-Film Evaporator: Poly(ethylene glycol)-400 (47 g) was added to the dark residue (89 g). EMF was distilled from this mixture on a wiped-film evaporator at 110°C, 4.7 mm Hg, and 400 rpm, yielding a yellow distillate (68 g) containing EMF (44 g, 44% molar yield from fructose), ethyl levulinate (20 g, ELA), and ethanol (5 g). NMR (δ, 1H): 9.54, (s, 0.8 H) EMF; 7.16, (d, 1.0 H), EMF; 6.46, (s, 1.0 H), EMF; 4.46, (s, 2.0 H), EMF; 4.05, (quartet, 1.0 H) ELA; 3.63, (quartet, 0.7 H), EtOH; 3.52, (quartet, 2.0 H), EMF; 2.68, (t, 1.1 H), ELA; 2.49, (t, 1.2 H), ELA; 2.12, (s,1.6 H), ELA; 1.17, (m, 5.6 H), ELA, EMF, EtOH.

## Claims

1. A method of preparing an R-oxymethylfurfural ether of hydroxymethylfurfural of the formula: 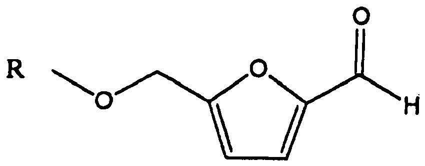 where R is selected from the group consisting of alkyl, cycloalkyl, and allyl, comprising:
combining a fructose source and an R-OH solvent to form a reaction mixture and contacting the reaction mixture with a solid acid catalyst bed in a chromatographic column;
heating the reaction mixture in the chromatographic column to a temperature and for a time sufficient to promote an acid-catalyzed dehydration reaction of fructose in the fructose source and to form R-oxymethylfurfural in a product mixture in the column; and
simultaneously chromatographically separating the product mixture in the column to at least partially separate the R-oxymethylfurfural from other components of the product mixture and elute an enriched fraction containing the R-oxymethylfurfural from the column.

2. The method of claim 1, wherein the R-OH solvent is ethanol and the R-oxymethylfurfural is ethoxymethylfurfural.

3. The method of claim 1, wherein the acid catalyst is an acidic ion exchange resin.

4. The method of claim 1, wherein the fructose source is a mixed fructose source.

5. The method of claim 1, wherein the method further includes distilling the enriched fraction to further purify the R-oxymethylfurfural.

6. The method of claim 1, wherein the yield of the R-oxymethylfurfural is in the range of 85-100%.

7. The method of claim 1, wherein the reaction mixture consists essentially of the fructose source and the R-OH solvent in contact with the solid acid catalyst.

8. The method of claim 1, wherein the column is eluted with the R-OH solvent of the reaction mixture.

9. The method of claim 1, wherein the reaction mixture consists essentially of the fructose source and the R-OH solvent in contact with the solid acid catalyst and the column is eluted with the R-OH solvent of the reaction mixture.

## Patentansprüche

1. Verfahren zur Herstellung eines R-Oxymethylfurfuralethers von Hydroxymethylfurfural der Formel 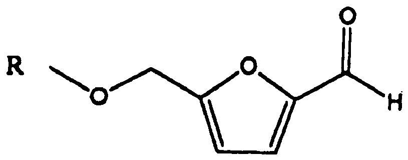 wobei R aus der Gruppe ausgewählt ist, die aus Alkyl, Cycloalkyl und Allyl besteht, umfassend:
Kombinieren einer Fructosequelle und eines R-OH-Lösungsmittels unter Bildung eines Reaktionsgemischs und In-Kontakt-Bringen des Reaktionsgemischs mit einem sauren Katalysator als Festbett in einer chromatographischen Säule;
Erhitzen des Reaktionsgemischs in der chromatographischen Säule auf eine ausreichende Temperatur und während einer ausreichenden Zeit, um eine säurekatalysierte Dehydratisierungsreaktion von Fructose in der Fructosequelle zu fördern und R-Oxymethylfurfural in einem Produktgemisch in der Säule zu bilden; und
gleichzeitig chromatographisches Auftrennen des Produktgemischs in der Säule, so dass R-Oxymethylfurfural wenigstens teilweise von anderen Komponenten des Produktgemisch abgetrennt wird und eine angereicherte Fraktion, die das R-Oxymethylfurfural enthält, von der Säule eluiert wird.

2. Verfahren gemäß Anspruch 1, wobei das R-OH-Lösungsmittel Ethanol ist und das R-Oxymethylfurfural Ethoxymethylfurfural ist.

3. Verfahren gemäß Anspruch 1, wobei der saure Katalysator ein saures Ionenaustauscherharz ist.

4. Verfahren gemäß Anspruch 1, wobei die Fructosequelle eine gemischte Fructosequelle ist.

5. Verfahren gemäß Anspruch 1, wobei das Verfahren weiterhin das Destillieren der angereicherten Fraktion zur weiteren Reinigung des R-Oxymethyl-furfurals umfasst.

6. Verfahren gemäß Anspruch 1, wobei die Ausbeute des R-Oxymethyl-furfurals im Bereich von 85-100% liegt.

7. Verfahren gemäß Anspruch 1, wobei das Reaktionsgemisch im Wesentlichen aus der Fructosequelle und dem R-OH-Lösungsmittel in Kontakt mit dem festen sauren Katalysator besteht.

8. Verfahren gemäß Anspruch 1, wobei die Säule mit dem R-OH-Lösungsmittel des Reaktionsgemischs eluiert wird.

9. Verfahren gemäß Anspruch 1, wobei das Reaktionsgemisch im Wesentlichen aus der Fructosequelle und dem R-OH-Lösungsmittel in Kontakt mit dem festen sauren Katalysator besteht und die Säule mit dem R-OH-Lösungsmittel des Reaktionsgemischs eluiert wird.

## Revendications

1. Procédé de préparation d'un éther R-oxyméthylfurfuralique d'hydroxyméthylfurfural de formule : 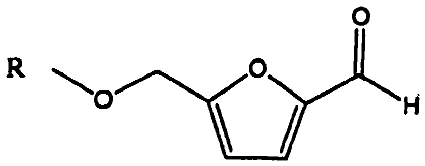 dans laquelle R est choisi dans le groupe constitué des groupes alkyle, cycloalkyle et allyle, comprenant les étapes consistant à :
- combiner une source de fructose et un solvant R-OH pour former un mélange réactionnel et mettre le mélange réactionnel en contact avec un lit de catalyseur acide solide dans une colonne chromatographique ;
- chauffer le mélange réactionnel dans la colonne chromatographique à une température et pendant une durée suffisantes pour favoriser une réaction de déshydratation à catalyse acide du fructose de la source de fructose et pour former du R-oxyméthylfurfural dans un mélange de produits, dans la colonne ; et
- séparer simultanément, par chromatographie, le mélange de produits dans la colonne pour séparer, au moins en partie, le R-oxyméthylfurfural des autres composants du mélange de produits et éluer une fraction enrichie contenant le R-oxyméthylfurfural de la colonne.

2. Procédé selon la revendication 1, dans lequel le solvant R-OH est l'éthanol et le R-oxyméthylfurfural est l'éthoxyméthylfurfural.

3. Procédé selon la revendication 1, dans lequel le catalyseur acide est une résine échangeuse d'ions acide.

4. Procédé selon la revendication 1, dans lequel la source de fructose est une source de fructose mixte.

5. Procédé selon la revendication 1, dans lequel le procédé comprend en outre l'étape consistant à distiller la fraction enrichie pour purifier davantage le R-oxyméthylfurfural.

6. Procédé selon la revendication 1, dans lequel le rendement du R-oxyméthylfurfural se situe dans la gamme de 85 % à 100 %.

7. Procédé selon la revendication 1, dans lequel le mélange réactionnel est essentiellement constitué de la source de fructose et du solvant R-OH en contact avec le catalyseur acide solide.

8. Procédé selon la revendication 1, dans lequel la colonne est éluée avec le solvant R-OH du mélange réactionnel.

9. Procédé selon la revendication 1, dans lequel le mélange réactionnel est essentiellement constitué de la source de fructose et du solvant R-OH en contact avec le catalyseur acide solide et la colonne est éluée avec le solvant R-OH du mélange réactionnel.
